(19) Europäisches Patentamt
European Patent Office
Office européen des brevets

(11) Publication number: 0 207 993
B1

(12) # EUROPEAN PATENT SPECIFICATION

(45) Date of publication of patent specification: 02.05.90

(21) Application number: 85900728.8

(22) Date of filing: 07.01.85

(86) International application number:
PCT/JP85/00001

(87) International publication number:
WO 86/04061 17.07.86 Gazette 86/17

(51) Int. Cl.5: C 07 D 493/04,
C 07 C 235/40, C 07 C 245/00,
C 07 C 323/37,
C 07 D 209/52,
C 07 D 251/04,
C 07 D 295/182,
C 07 D 311/94, C 08 G 69/00,
C 08 G 69/40, C 08 G 69/42

(54) ## OPTICALLY ACTIVE, ANTI HEAD-TO-HEAD COUMARIN DIMER-POLYAMIDES.

(43) Date of publication of application:
14.01.87 Bulletin 87/03

(45) Publication of the grant of the patent:
02.05.90 Bulletin 90/18

(84) Designated Contracting States:
BE DE FR GB NL

(56) References cited:
Tetrahedron Letters, vol. 24. No. 48, P5381-5384 (1983)

Chemical Abstracts. Vol. 101. No. 23, 210922g (1984)

Chemical Abstracts. Vol. 76. No. 25, 152890x (1972)

Chemical Abstracts, Vol. 99, No. 16, 123078h

Chemical Abstracts, Vol. 98, No. 21, 179161b (1983)

Chemical Abstracts, Vol. 97. No. 8, 56330C (1982)

Chemical Abstracts. Vol. 79, No. 2, 5665m (1973)

(73) Proprietor: HASEGAWA, Masaki
26-2, Miyamae 2-chome
Suginami-ku Tokyo 168 (JP)

(72) Inventor: SAIGOU, Kazuhiko
1-37, Asahicho 2-chome
Souka-shi Saitama-ken 340 (JP)
Inventor: YONEZAWA, Noriyuki
203, 18-12, Hongo 5-chome
Bunkyo-ku Tokyo (JP)
Inventor: KANOE, Toshio
52, Morishita
Fuji-shi Shizuoka-ken 416 (JP)
Inventor: SEKIMOTO, Kazuhiro
66-21, Ohaza-Minamida
Izumi, Kimitsu-shi Chiba-ken 299-11 (JP)

(74) Representative: Livsey, Gilbert Charlesworth Norris et al
HYDE, HEIDE & O'DONNELL 146 Buckingham Palace Road
London SW1W 9TR (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 207 993 B1

(56) References cited:
Chemical Abstracts. Vol. 101. No. 25, 229551g (1984)

Chemical Abstracts. Vol. 99. No. 15, 121855k (1983)

Chemical Abstracts. Vol. 78. No. 17, 110192g (1973)

**Description**

Technical Field

The present invention relates to a novel optically active polyamide which corresponds to a derivative of an optically active, anti head-to-head coumarin dimer of the following formula (I) or (II).

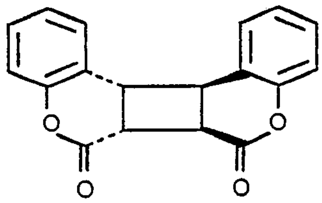

(I)

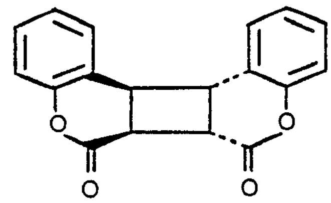

(II)

These two optically active isomers are hereinafter referred to as the (+)-dimer and the (−)-dimer, respectively.

Technical Background of the Invention

The anti head-to-head coumarin dimer can be obtained by photodimerizing coumarin.

This compound is optically inactive, i.e. a (±)-dimer.

We have found that when the optically active isomer, i.e. (+)- or (−)-dimer, is reacted with a diamino compound, a novel optically active polymer can be obtained. The present invention is based on these findings.

Disclosure of the Invention

The present invention utilises an optically active, anti head-to-head coumarin dimer of the following formula (I) or (II):

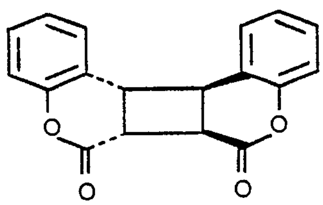

(I)

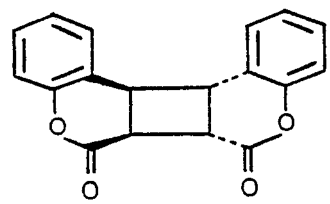

(II)

Further, the present invention utilises a process for preparing an optically active, anti head-to-head coumarin dimer of the following formula (I) or (II):

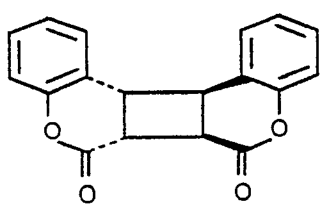

(I)

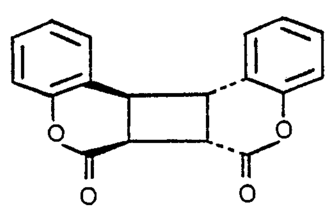

(II)

characterized by separating a diamide derived from the racemic, anti head-to-head coumarin dimer and an optically active amine into diastereomers, deaminating each of them and ring-closing the product.

The method for obtaining the optically active dimer is characterized in that a diamide derived from the racemic, anti head-to-head coumarin dimer and an optically active amine is resolved into diastereomers, each diastereomer is deaminated and the product is ring-closed. The method involves steps represented by the following formulae:

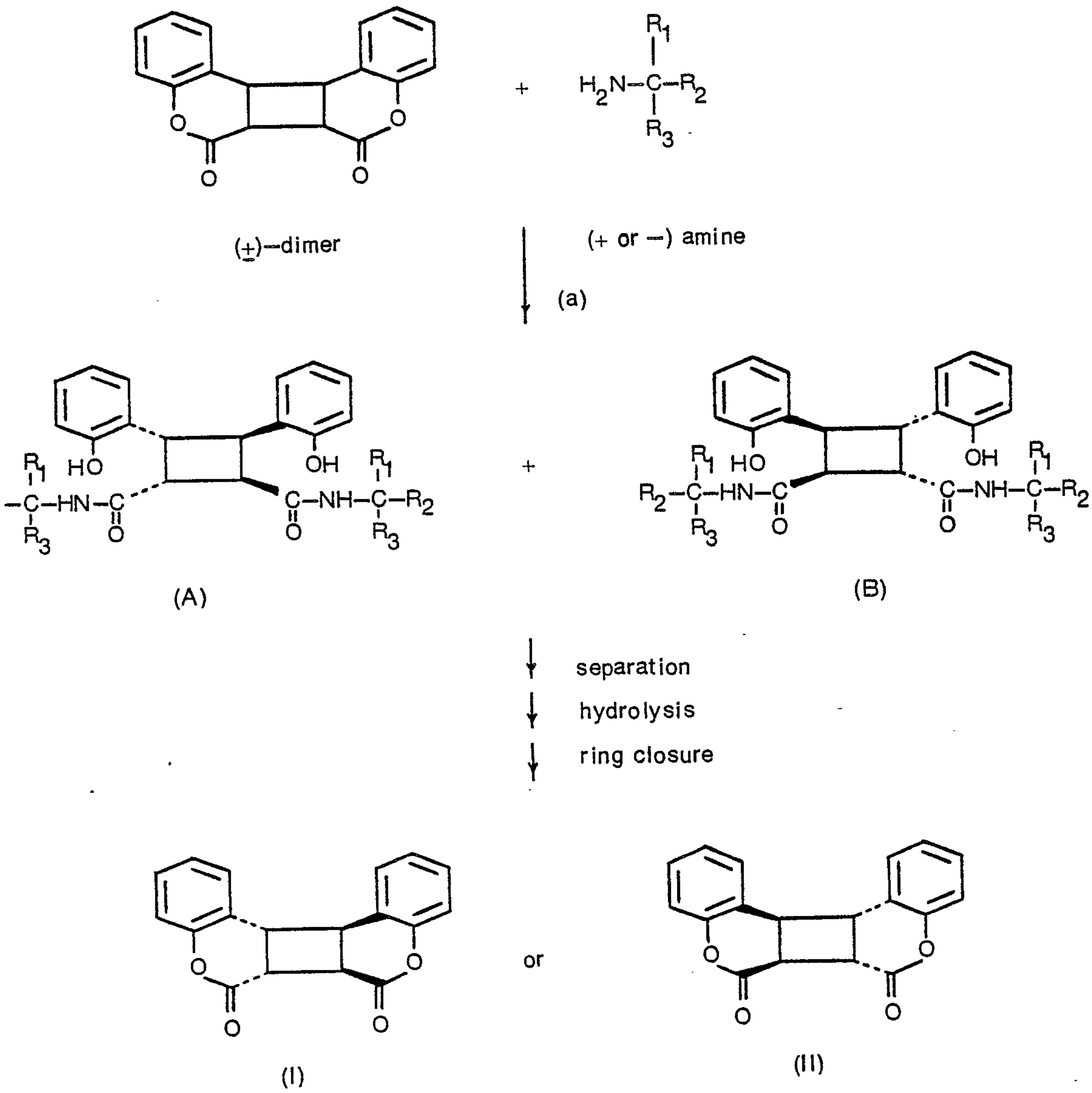


(A) (B)

(I) or (II)

The amidation reaction (a) readily proceeds when the dimer is mixed and reacted with the optically active amine in a solvent such as dioxane.

The amides (A) and (B) are diastereomers and differ in solubility from each other. Therefore, when the reaction is carried out in an appropriate solvent under specified conditions, it is possible to precipitate and separate only one diastereomer as the reaction proceeds. For example, when (−)-1-phenylethylamine is used as the amine, the amide derived from the (+)-dimer is difficulty soluble in dioxane, while the one derived from the (−)-dimer is easily soluble.

Amino acid derivatives, alkaloid derivatives, etc. can also be used as the amine in the present invention.

The amide which is separated and purified by an appropriate method such as recrystallization can be hydrolyzed by any of conventional methods. For example, a method using alcoholic HCl is preferred.

The hydrolyzate is ring-closed by a conventional lactonization method as such or, if necessary, after purification. For example, the ring closure is readily effected by heating in acetic acid under reflux to form the (+)- or (−)-dimer.

The polyamide of the present invention is different in optical properties as well as solubility, thermal properties, etc. from the corresponding racemic polyamide.

The polyamide of the present invention contains highly reactive phenol groups in the molecule and the optical activity has a characteristic originating from the cyclobutane ring, so that the polyamide itself or its derivative which has a substituted phenol group or in which part of the cyclobutane rings are cleaved is a material suitable for use in asymmetric synthesis, optical resolution analysis, etc.

The optically active polyamides of the present invention can be represented by the following formulas:

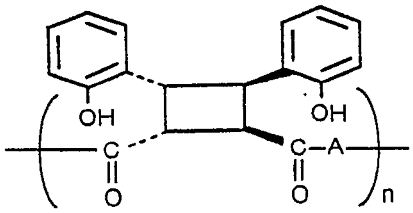

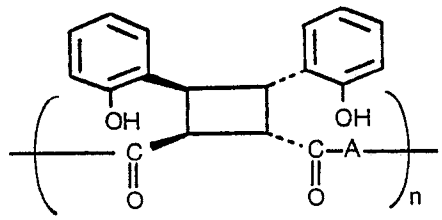

wherein A is a residue formed by removing one active hydrogen atom from each of two amino groups of a primary or secondary diamine. The diamine may be saturated or unsaturated. It may be a mixture of two or more diamines or modified with a triamine or the like.

The polymer chain is usually terminated with either a hydrogen atom or the above dimer (I) or (II), and sometimes with an impurity. The polymers of the present invention are polycondensates, and n in the above formula is at least 2, preferably from 2 to 1000.

Examples of residue A in the above formula are as follows:

$-HN(CH_2)_2-NH-$ , $-HN-(CH_2)_6-NH-$ , 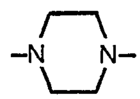 , 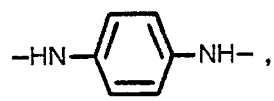 ,

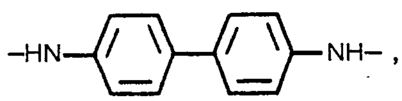 , 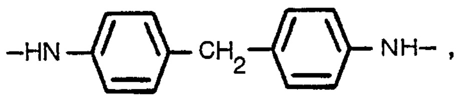 ,

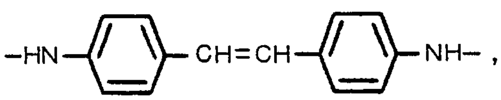 , 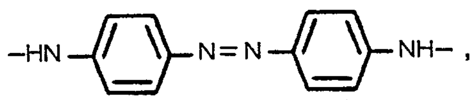 ,

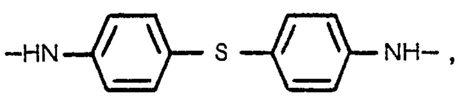 , 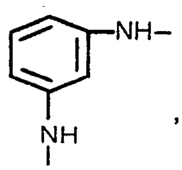 , 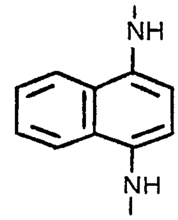 ,

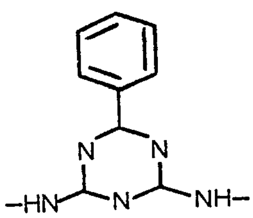 , 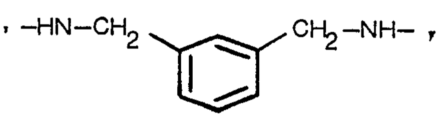 , 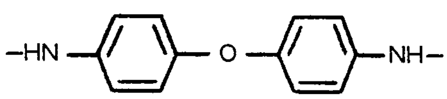

The optically active polyamide of the present invention can be easily obtained by reacting the (+) or (−) anti head-to-head coumarin dimer with the corresponding diamine. Examples of preferred diamines are hexamethylene diamine, m-xylylenediamine, 4,4′-diaminophenyl ether and piperazine. Usually, the reaction proceeds in the absence of a specific catalyst. Preferably, both reactants in equimolar quantities are reacted in an aprotic polar solvent. Preferred examples of the solvents include dimethylacetamide, dioxane, methylpyrrolidone, dimethyl sulfoxide and hexamethyl phosphoric triamide.

When the coumarin dimer and the diamine are used in a molar ratio of 1:1 in the above polymerization reaction, a polymer having a relatively high degree of polymerization can be obtained, while when the molar ratio is changed or a monofunctional compound such as a monoamine is added, a polymer having a relatively low degree of polymerization can be obtained.

The following examples will further illustrate the present invention. Examples 1 to 3 relate to preparation of precursors of the optically active polyamides of the invention. Examples 4 to 8 relate to preparation of the polyamides.

58.46 g of the (±)-dimer was dissolved in 850 ml of dioxane. 48.47 g of (−)-1-phenylethylaine was added dropwise thereto while stirring over a period of 30 min. After stirring for 12 hr, the precipitate was separated by filtration, washed with dioxane and dried at 60°C in vacuo to afford 42.29 g of a crystal.

The product was diamine (A) derived from the (+)-dimer and the yield was 39.5%.

After the filtrate was concentrated, 150 ml of acetone and 150 ml of methanol were added thereto and the mixture was dissolved by heating left to stand at 5°C for 12 hr.

The precipitated crystal was dried to afford 35.54 g (yield 33.3%) of the diamide (B) derived from the (−)-dimer.

The properties of these diamides A and B are shown in Table 1.

TABLE 1

| | Melting point (°C) | $[\alpha]_D^{21}$ *) | Elemental analysis (calc.d) (%) |
|---|---|---|---|
| Diamide A | 237—238 | −19.0° | C 76.54 (76.38)<br>H 6.27 (6.40)<br>N 5.17 (5.23) |
| Diamide B | 246—247 | −177.3° | C 76.66 (76.38)<br>H 6.16 (6.40)<br>N 5.19 (5.23) |

*) c 0.5, 99% methanol

Example 2

5,346.5 mg of the diamide of the (−)-dimer was added to a mixture of 100 ml of 99% ethanol and 50 ml of concentrated hydrochloric acid, and the mixture was refluxed.

After 20 hr, the reflux was terminated and the mixture was concentrated to remove ethanol. 200 ml of water was added thereto and the mixture was extracted three times with ethyl acetate (each 150 ml).

After ethyl acetate was removed, 150 ml of acetic acid was added and the mixture was refluxed.

The resulting material was developed on a silica gel column (Wakogel C-200, 4 ϕ × 15 cm) eluting with 500 ml of benzene and then 500 ml of a mixture of benzene and ethyl acetate (90:10) successively to afford 2451.9 mg (yield 83.9%) of the crude (−)-dimer crystal. This crude crystal was recrystallized from a solvent mixture of ethyl acetate and hexane (2:3) to afford 1.92 g (yield 65.7%) of the (−)-dimer.

Example 3

The diamide of the (+)-dimer was treated in a similar manner to that described in Example 2 to afford the (+)-dimer in a yield of 63.4%.

The properties of these (+)- and (−)-dimers as well as those of the (±)-dimer as a reference are shown in Table 2.

TABLE 2

| | Melting point (°C) | $[\alpha]_D^{21}$ *) | $[\alpha]_{435}^{21}$ *) |
|---|---|---|---|
| (+)-Dimer | 168—169 | +9.0° | +66.0 |
| (−)-Dimer | 168.5—169 | −9.0 | −65.8 |
| (±)-Dimer | 187.5—189 | — | — |

*) c 1.0, benzene

Example 4

0.2552 g (0.9 mmol) of the (−) anti head-to-head coumarin dimer and an equimolar quantity of p-phenylenediamine were dissolved in 1.7 ml of dimethylacetamide and left to stand at 80°C for 24 hr.

After the completion of the reaction, the product was added dropwise to methanol to afford a polymer (yield 87%).

The resulting polymer had a reduced viscosity of 0.36 (0.3 g/dl in dimethylacetamide at 30°C) and the optical activity was $[\alpha]_D$ = −89.4° and $[\alpha]_{435}$ = −260.0° (0.5 g/100 ml in dimethylacetamide).

Example 5

The procedure of Example 4 was repeated except that 0.8 mmol of hexamethylenediamine was used as the diamine and the reaction was carried out in 1.6 ml of dimethylacetamide.

The yield and the properties of the product are shown in Table 3.

Example 6

2.1 mmol of m-xylylenediamine was used and the reaction was carried out in 4.3 ml of dimethylacetamide in a similar manner to that described above.

The yield and the properties are shown in Table 3.

Example 7

1 mmol of 4,4′-diaminodiphenyl ether was used and the reaction was carried out in 2.0 ml of diemthylacetamide in a similar manner to that described above.

The yield and the properties are shown in Table 3.

Example 8

1 mmol of piperazine was used and the reaction was carried out in 2.1 ml of dimethylacetamide in a similar manner to that described above.

The yield and the properties are shown in Table 3.

TABLE 3

| Example No. | Diamine | Yield (%) | Reduced[a] Viscosity | $[\alpha]_D$ | $[\alpha]_{435}$ |
|---|---|---|---|---|---|
| 5 | hexamethylenediamine | 86 | 0.68 | $-59.0^{\circ}$[b] | $-160.9^{\circ}$[b] |
| 6 | m-xylylenediamine | 86 | 0.63 | $-26.8^{\circ}$[c] | $-79.3^{\circ}$[c] |
| 7 | 4,4′-diaminodiphenyl ether | 65 | .31 | $-91.6^{\circ}$[d] | $-266.7^{\circ}$[d] |
| 8 | piperazine | 97 | 0.51 | $+30.6^{\circ}$[c] | $+45.4^{\circ}$[c] |

Note:
(a) in dimethylacetamide, 0.3 g/dl, 30°C
(b) in dimethylacetamide, 0.49 g/dl
(c) in dimethylacetamide, 0.5 g/dl
(d) in dimethylacetamide, 0.51 g/dl

**Claims**

1. An optically active polyamide of the formula (III) or (IV):

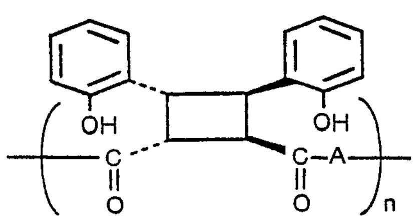

(III)

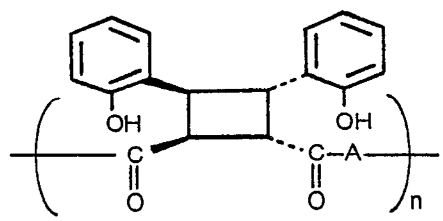

(IV)

wherein A is a residue formed by removing one active hydrogen atom from each of two amino groups of a primary or secondary diamine.

2. A process for preparing an optically active polyamide of the formula (III) or (IV):

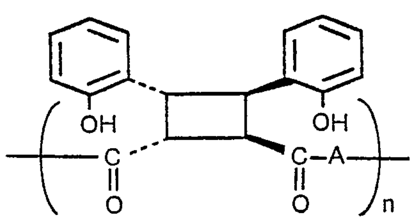

(III)

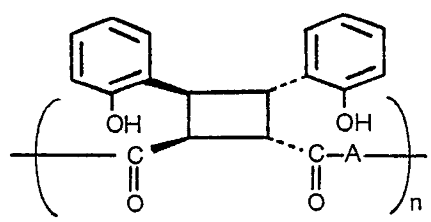

(IV)

wherein A is a residue formed by removing one active hydrogen atom from each of two amino groups of primary or secondary diamine; which comprises reacting an optically active, anti head-to-head coumarin dimer with a primary or secondary diamine.

**Patentansprüche**

1. Optisch aktives Polyamid der Formel (III) oder (IV)

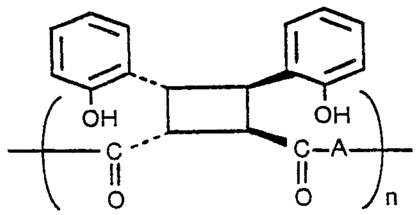

(III)

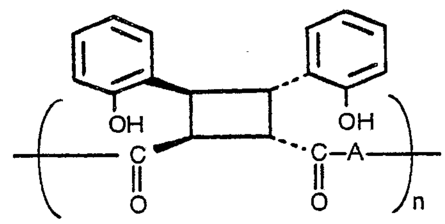

(IV)

worin A eine Rest ist, der durch Entfernen eines aktiven Wasserstoff-Atoms von jeder der zwei Amino-Gruppen eines primären oder sekundären Diamins gebildet ist.

2. Verfahren zur Herstellung eines optisch aktiven Polyamids der Formel (III) oder (IV)

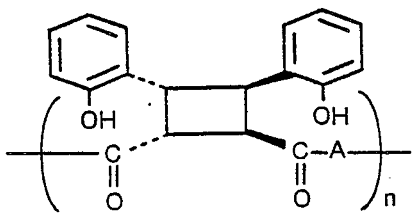

(III)

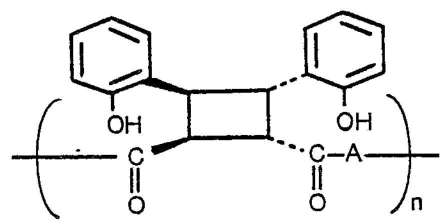

(IV)

worin A ein Rest ist, der durch Entfernen eines aktiven Wasserstoff-Atoms von jeder der zwei Amino-Gruppen eines primären oder sekundären Diamins gebildet ist, umfassend die Umsetzung eines optisch aktiven Anti-Kopf-zu-Kopf-Cumarins-Dimers mit einem primären oder sekundären Diamin.

**Revendications**

1. Un polyamide optiquement actif de la formule (III) ou (IV):

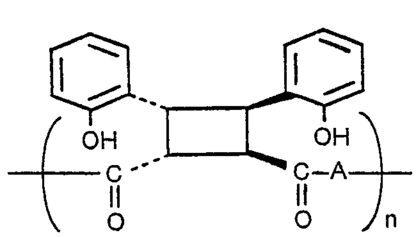

(III)

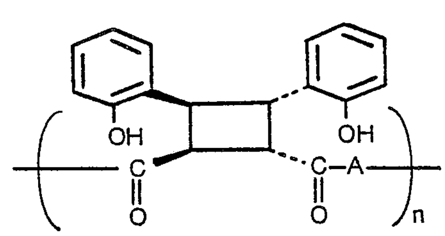

(IV)

où A est un résidu formé en enlevant un atome d'hydrogène actif de chacun des deux groupes amino d'une diamine primaire ou secondaire.

2. Un procédé pour préparer un polyamide optiquement actif de la formule (III) ou (IV):

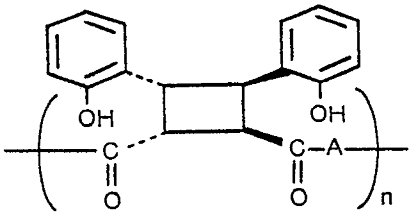

(III)

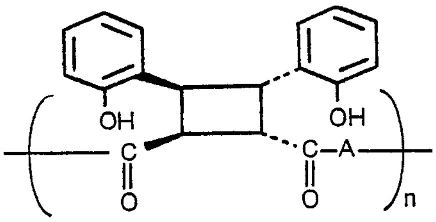

(IV)

où A est un résidu formé en enlevant un atome d'hydrogène actif de chacun deux groupes amino d'une diamine primaire ou secondair; qui consiste à faire réagir un dimère de coumarine anti-tête-à-tête optiquement actif avec une diamine primaire ou secondaire.